# EUROPEAN PATENT APPLICATION

(11) **EP 1 172 378 A1**
(43) Date of publication of application: **16.01.2002**
(21) Application number: 01114825.1
(22) Date of filing: 28.06.2001
(51) Int. Cl.: C07K 16/18, A61K 39/395, C07K 1/22, G01N 33/68, A61P 25/28

(54) **Human beta-amyloid antibody and use thereof for treatment of alzheimer's disease**

(30) Priority: 12.07.2000 EP 00115141
(71) Applicant: Dodel, Richard, Dr., 35039 Marburg (DE); Du, Yansheng, Dr., Carmel, Indiana 46033 (US)
(72) Inventor: Dodel, Richard, Dr., 35039 Marburg (DE); Du, Yansheng, Dr., Carmel, Indiana 46033 (US)
(74) Representative: Schwabe - Sandmair - Marx

(57) **Abstract**

The present invention provides according to the first aspect thereof a human anti-β-amyloid antibody obtained by purification from a human IgG-containing bodyfluid by Aβ-affinity chromatography. In a second aspect the invention provides a method of purification of an anti-Aβ-amyloid antibody, said method comprising the steps of obtaining a human IgG-containing bodyfluid, subjecting the bodyfluid obtained to an Aβ-affinity chromatography, and recovering the purified anti-Aβ antibody from the chromatography medium. Finally the invention provides for use of the above anti-Aβ antibody for diagnosing and/or treating amyloid associated diseases, especially Alzheimer's disease and for a pharmaceutical composition comprising said antibody for treatment of Alzheimer's disease.

## Description

The present invention relates to a human β-amyloid antibody, a method of purification thereof and the use of this β-amyloid antibody in treatment of amyloid associated diseases, especially Alzheimer's Disease.

### BACKGROUND

Alzheimer's disease is a progredient disease initially manifesting itself with partial amnesia, and later restlessness, dysorientation, aphasia, agnosia or apraxia (cognitive decline), dementia and sometimes euphoria or depressions. The disease typically starts at 40 to 90 years of age and predominantly affects females. As to its occurance, estimations are about 5 % of the population above 65 years age. Alzheimer thus constitutes a major problem in industrialised countries.

In Alzheimer's disease brain region-specific amyloid deposition is a key neuropathological feature which is accompanied by astrogliosis, microgliosis, cytoskeletal changes, and synaptic loss. These pathological alterations are thought to be linked to the cognitive decline and dementia which defines the disease. These neuritic depositions or plaques and neurofibrillary tangles comprise the major neuropathological changes associated with Alzheimer's disease. Although other neuropathological changes have been linked to Alzheimer's disease, evidence indicates that they are as well somehow related to the classical lesions.

Neuritic plaques are spherical, multicellular lesions that are usually found in moderate or large numbers in limbic structures and association neocortex. The plaques contain extracellular deposits of β-amyloid protein (Aβ) that include abundant amyloid fibrils intermixed with non-fibrillar forms of this peptide. The major protein constituent of plaques is the β-amyloid protein (Aβ). Neuritic plaques have degenerating axons and dendrites within and intimately surrounding the plaque. Such plaques also contain variable numbers of activated microglia that are often situated within and near the fibrillar amyloid core, as well as reactive astrocytes surrounding the core.

The major constituent of the plaque, the β-amyloid protein, arises from a larger precursor protein, the amyloid precursor protein (APP). The amyloid precursor protein (APP) refers to a group of ubiquitously expressed proteins whose heterogeneity arises from both alternative splicing and posttranslational processes. Cleavage of APP in its COOH-terminal region in the transmembrane domain by β-secretase and γ-secretase results in the formation of the β-amyloid protein.

Aβ is secreted continously by normal cells and can be detected as a circulating peptide in the plasma and cerebrospinal fluid (CSF) of healthy humans. In Alzheimer's disease it is thought that increased production of Aβ and/or a decreased metabolism of Aβ may lead to plaque deposition and consecutively to the neuropathological changes associated with Alzheimer's disease. Evidence for the role of Aβ in Alzheimer's disease include the observation that misssense mutations in the APP have been found to be the cause of familial Alzheimer disease cases.

Several endogenous substrates, including apolipoprotein E have been shown to be asso-ciated with plaque formation. In transgenic mice APP^{V717F} (PDAPP) the lack of the apolipoprotein E gene (apoE-knock-out mice) results in the absence of amyloid plaque deposition (Games et al., Nature 1995; Bales et al., Nat Genet 1997). These transgenic mice (PDAPP) normally develop amyloid plaques in an age-dependent manner starting at three months of age.

Schenk and coworkers (Schenk et al, Nature 400:173, 1999) investigated the plaque burden in the PDAPP-mice following an immunization treatment. PDAPP-mice were immunised with pre-aggregated Aβ for different time periods using Freud's adjuvans. Plaque deposition in these mice decreased significantly following the immunization treatment. Sham-mice did not show a decrease in plaque deposition.

Treatment of APP^{V717F} transgenic mice with antibodies raised against Aβ was also reported to attenuate amyloid plaque formation, neuritic dystrophy and astrogliosis in younger mice as well as to decrease plaque burden in older mice. However, the finding could not be verified in other mice.

Despite of the above knowledge no therapy for amyloid associated diseases, especially Alzheimer's disease is available up to today. However, an effective therapy for Alzheimer's disease would be highly desirable because of its broad spread occurance.

It is therefore an object of the present invention to provide such therapy of and/or means for diagnosing amyloid associated diseases, especially Alzheimer's disease.

### SUMMARY OF THE INVENTION

The above object can be solved by a human anti-β-amyloid antibody and a pharmaceutical composition comprising the same as stipulated in the appending claims.

More in detail the present invention according to the first aspect thus provides a human anti-β-amyloid antibody obtained by purification from a human IgG-containing bodyfluid by Aβ-affinity chromatography.

In a second aspect the invention provides a method of purification of an anti-Aβ-amyloid antibody, said method comprising the steps of obtaining a human IgG-containing bodyfluid, subjecting the bodyfluid obtained to an Aβ-affinity chromatography, and recovering the purified anti-Aβ antibody from the chromatography medium.

Finally the invention provides for use of the above anti-Aβ antibody for diagnosing (with a special developped ELISA) and/or treating amyloid associated diseases, especially Alzheimer's disease and for a pharma-ceutical composition comprising said antibody for treatment of amyloid associated diseases, especially Alzheimer disease and manufacture thereof.

### DETAILED DESCRIPTION OF THE INVENTION

The applicants have now found that naturally-occurring Aβ antibodies exist in biologically relevant fluids i.e. CSF and plasma, and that levels of these antibodies differ between normal age-matched healthy controls and AD patients. Based on these findings it was concluded and then supported by experiments that the antibody can be used for diagnosis and treatment of amyloid associated diseases and especially of Alzheimer's disease. In the context of this specification the terms "anti-Aβ antibodies" and "Aβ antibodies" are used interchangeably to designate the antibody of the invention.

In lumbar CSF samples which included 49 age-matched non-demented individuals with no family history of cognitive impairment and 60 individuals with confirmed AD, detection of CSF Aβ antibody levels was determined utilising an ELISA assay in which the Aβ peptide was used as the capture ligand (see below).

Human anti-Aβ antibody was detected in CSF samples in both of the populations studied. It was confirmed that the Aβ antibody activity detected by the ELISA represents antibodies specific to Aβ by absorbing the activity with protein-A Agarose, and Aβ₁₋₄₀ or Aβ₁₋₄₂. However, no interaction was found between this Aβ-antibody and Aβ₄₀₋₁ or unrelated neuropeptides such as neuropeptide F or neuropeptide Y. The mean level of Aβ antibody in the Alzheimer's disease group was 30% lower than controls (control: 370±39, AD: 276±27; p < 0.05, one way ANOVA).

These data demonstrate that an antibody directed against Aβ (anti-Aβ antibody or short: Aβ antibody) is present in physiologically relevant concentrations in human fluids, like CSF and serum. Antibody titres are significantly higher in control subjects than AD patients. The generation of naturally occurring Aβ-antibodies and subsequent Aβ / antibody complex formation, may be involved in the normal clearance of Aβ peptide(s), which serves to reduce Aβ deposition and neuritic plaque formation.

The lower titres of Aβ antibody found in more than 50% of the AD patients investigated in this study compared to controls suggest that reduced Aβ antibody generation and/or complex formation contributes to an abnormal (i.e. reduced) clearance function. Similar clearance problems may occur in other neurodegenerative diseases or amyloid associated diseases such as primary and secondary amyloidoses. The present invention thus pertains to treatment and diagnosis of these other amyloid associated diseases as well.

Based on the above hypothesis the treatment with antibodies against Aβ i.e. Aβ antibodies is a new strategy to treat diseases associated with amyloid deposition. These treatments include the increase of Aβ-antibody levels by using immunoglobulins (IgG), preferably human IgG with high titres of Aβ antibodies or using anti-Aβ antibodies purified from human IgG containing fluids. The present invention also encompasses use of antibody fragments (Fab etc.) as long as complex formation can be achieved.

Thus, the present invention relates to a human anti-β-amyloid antibody (Aβ-antibody) obtained by purification from a human IgG-containing bodyfluid by Aβ-affinity chromatography. Preferably the human anti-Aβ antibody belongs to the class of immunoglobulines G (IgG) and does not recognise Aβ₄₀₋₁, neuropeptide F, neuropeptide Y, and Amylin, and specifically recognises one or more of Aβ₁₋₄₀, Aβ₁₋₄₂, and Aβ₂₅₋₃₅, and preferably recognises all of Aβ₁₋₄₀, Aβ₁₋₄₂, and Aβ₂₅₋₃₅.

According to a second embodiment the present invention relates to a method of purification of an anti-Aβ-antibody comprising the steps of obtaining a human IgG-containing bodyfluid, subjecting the bodyfluid obtained to an Aβ-affinity chromatography, and recovering the purified anti-Aβ antibody. Preferably the IgG-containing bodyfluid is a fluid selected from the group consisting of cerebrospinal fluid, plasma and urine, all of them obtained from one or more human beings (pooled samples).

Furthermore, it is preferred that the Aβ-affinity chromatography is carried out by an Aβ-affinity column, obtained by conjugating Aβ₁₋₄₀ onto Sepharose 4B, elution with elute buffer at pH 1.5 to 2.5 at 4°C using an FPLC system.

The present invention also relates to the use of the above anti-Aβ antibody and/or the use of an IgG containing, preferably IgG enriched fluid for diagnosing and/or treating amyloid associated diseases, especially Alzheimer's disease. Preferably the use is for treatment of amyloid associated diseases, especially Alzheimer's disease.

According to another embodiment there is provided a pharmaceutical composition comprising the anti-Aβ antibody of the present invention. A pharmaceutical composition of the invention comprises the anti-Aβ antibody and is preferably for parenteral administration, e.g. by i.v., i.m. or i.c. injection. It may comprise conventional carriers. A preferred dosage for administration is in the range of 0.001 to 3 g/kg body weight per day, a more preferred dosage for administration being in the range of 0.01 to 0.4 g/kg body weight per day.

The experimental work forming the basis of the present invention was carried out using the following materials and methods:

**Aβ antibody ELISA: 1** mg Aβ₍₁₋₄₀₎ is dissolved in 2ml H₂O. Then add up to 200 ml coating buffer (1.7mM NaH₂PO₄*H₂O; 98 mM Na₂HPO₄*7H₂O, 0.05% sodium azide; pH 7.4). Add 100 µl/well of coating buffer overnight at 4°C. Remove coating buffer and block plate with blocking buffer for 80 min. (blocking buffer 1: 0.25% casein in PBS, 0.05% sodium azide, pH=7.4). Wash plate 3 times with washing buffer (1xPBS/0.05% Tween-20). Load samples overnight at 4°C. Remove samples and wash plate 3 times. Add monoclonal anti-human biotinylated IgG in blocking buffer 1 for 1 h. Wash 3 times with washing buffer. Load antibody against biotin conjugated with horse radish peroxidase for 1h. Wash four times and add TMP for 10 min, then add H₂SO₄ (1N) to stop reaction and read at a plate reader at 450 nm.

**β-Amyloid-ELISA:** For the measurement of Aβ a commercially available kit for Aβ₁₋₄₂, Aβ₁₋ ₄₀ and Aβ₁₋₅ was used.

**Cerebrospinal fluid (CSF) and plasma:** lumbar CSF and plasma were collected following standard clinical procedures after informed consent of the patients.

**Criteria for the diagnosis of Alzheimer's disease:** All normal controls had no significant decline or impairment in cognition on clinical examination. They had no history or evidence of neurological disease with potential to affect cognition and no deficits in their ability to adequately perform activities of daily living (ADLs). All AD patients had a clinical examination, including neuropsychological testing, to document deficits in cognition and ADLs, laboratory studies and a neurological examination to exclude reversible causes of dementia. All patients met ICD-10 criteria for dementia as well as NINCDS-ADRDA criteria for probable or possible AD.

### DESCRIPTION OF THE DRAWINGS:

### Figure 1:

Aβ-antibody has been identified in the CSF from Alzheimer's disease patients (AD) and control individuals. Levels of Aβ antibodies in CSF from AD patients were reduced by 30% when compared to age-matched control subjects (p<0.05, one way ANOVA)

### Figure 2:

1 Aβ antibody unit = 10 antibody titres.
1 ml of CSF was incubated with 1, 10, or 100 µl of protein A conjugated with agarose bead (Sigma P-7786) overnight at 4 °C (Pa). After removing protein A, 290 µl of CSF were used to determine the titre of antibody. 1 ml of CSF was also incubated with Aβ₁₋₄₀, Aβ₁₋₄₂, Aβ₂₅₋₃₅ (1 mg Aβ was dissolved in 0.9 ml of deionised H₂O). 0.8-20 µL of Aβ was used for overnight incubation (at 4 °C) with CSF. 290 ml of CSF was then used for determination.
PE 1-100 µL: Protein A precipitates (1, 10, or 100 µl) from CSF sample was incubated with 100 µl of PBS (pH 2.5). The recovered solution was used for titre determination. The antibody titre is defined as the dilution of antibody that gives a half-maximal binding to antigen.
(Pa: Protein A; Aβ₁₋₄₀: β-Amyloid 1-40; Aβ₁₋₄₂: β-Amyloid 1-42; Aβ₂₅₋₃₅: β-Amyloid 25-35; PE: elute from protein A precipitates)

### Figure 3:

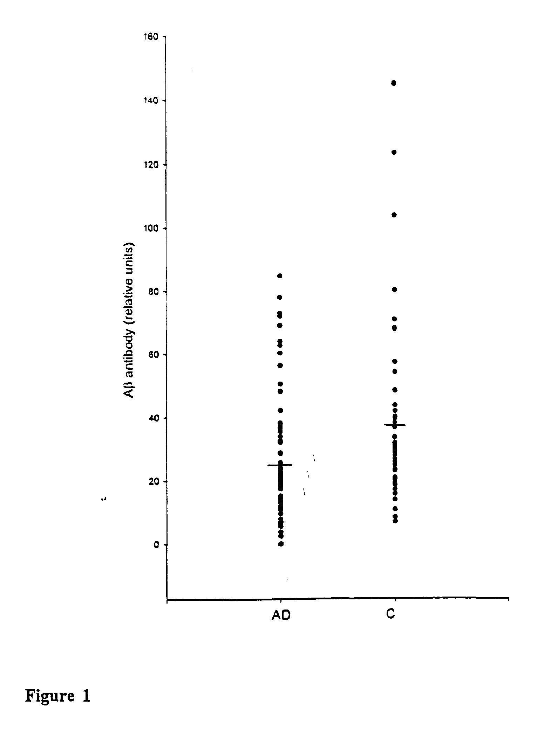

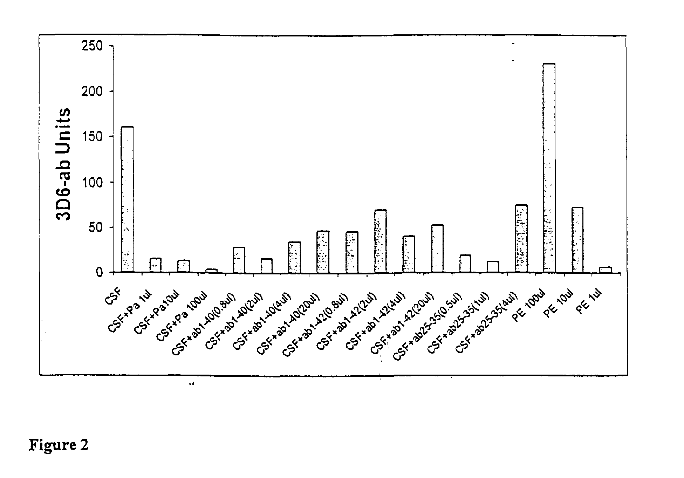

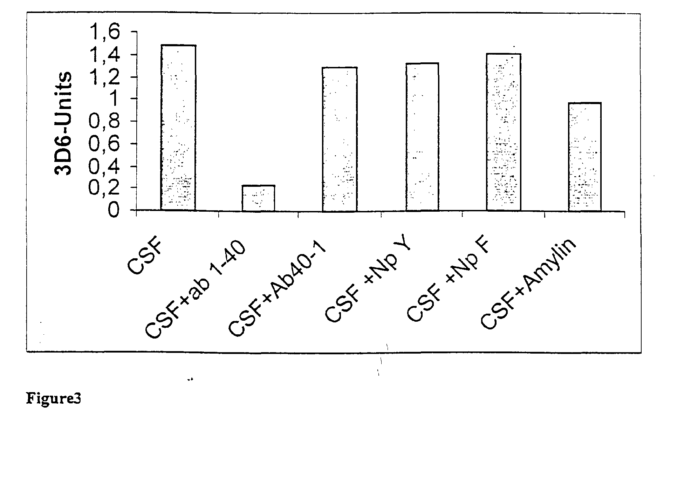

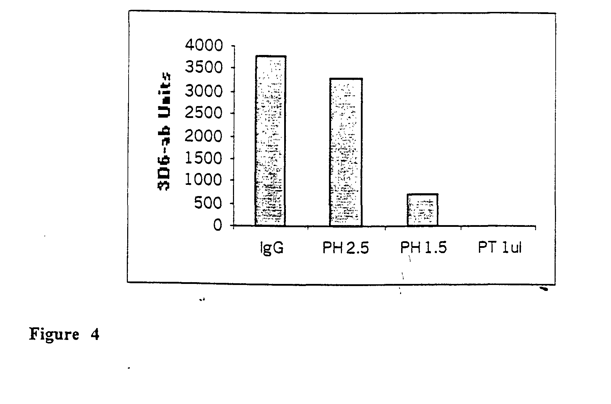

Same condition as in Figure 2. Only Aβ₁₋₄₀=2 µl. Aβ₄₀₋₁=2 µl. Neuropeptide F and neuropeptideY (2 µl), Amylin (2 µl).

### Figure 4:

Purification of anti-Aβ antibodies by using Aβ affinity column.
After 250 g immunoglobulin (IgG) pass through the Aβ affinity column, 10 ml of elute buffer (pH2.5) was used to elute Aβ antibody. Then another 10 ml of elute buffer (pH1.5) was used to elute the remainder of antibodies. After ELISA detection, significant amount of Aβ antibody was detected in pH 2.5 elute buffer. IgG = immunoglobulin 100 µl. PH2.5, 1.0: elute antibodies by using pH2.5 and then pH1.5 buffers from affinity column: 100 µl. PT: IgG pass through Aβ affinity column, equal to 100 µl of IgG. Most anti-Aβ antibody elute from column by pH 2.5. Column: 3 mg of Aβ₁₋₄₀ was conjugated into Sepharose 4B (Pharmacia, 5 ml). Purification by using Pharmacia FPLC system at 4 degree. 1 Aβ antibody unit = 10 antibody titres. (Elute buffer: 50 mM glycine, 150 mM NaCl, pH 2.5).

### Figure 5:

Concentration of β-Amyloid in the CSF before treatment with immunoglobulins and 7-12 days and 4 weeks after treatment, respectively. Measurements were done as described in Example 2.

The following examples are given for illustration purposes only and are not intended to limit the scope of the invention.

### Example 1: Treatment of AD patients by infusion of human IgG immunoglobulins or anti-Aβ antibodies from human IgG.

As an example as to the therapy regimen 5 - 30 g (1 - 5 days) of IgG immunoglobulins (commercially available) or a corresponding amount of purified anti-Aβ antibody are administered parenterally to the patient by the i.v. route. Levels of β-Amyloid, tau-protein as well as Aβ-antibody are measured in the serum and CSF before and following the respective dose of IgG immunoglobulins for therapy control. The goal is to decrease β-amyloid concentration in the CSF and by that decrease the plaque burden in Alzheimer's disease and alleviate the neuropsychiatric and neuropsychological defects in Alzheimer's disease. This treatment introduces a new therapeutic approach to Alzheimer's disease.

### Example 2: Effect of i.v. immunoglobulins on the Concentration of β-Amyloid in the CSF

In this example the effect of the application of i.v. immunoglobulins (Octagam® , Polyglobulin® ) on the concentration of β-Amyloid in the CSF is investigated.

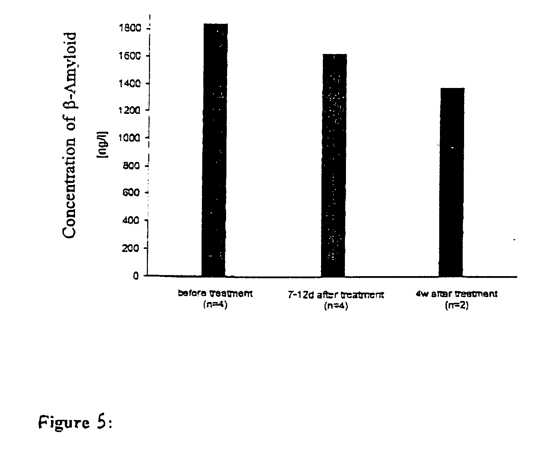

Four patients suffering from different neurological disorders (Guillain-Barre-Syndrome; chronic inflammatory demyelinating neuropathy, CIDP) were included in this study. Lumbar CSF was withdrawn before starting treatment with i.v. immunoglobulins. After 7 to 12 days and 4 weeks an additional lumbar puncture was performed. The withdrawal of CSF was performed during regular investigations. Patients were treated with i.v. immunoglobulins for 3 - 5 days with 0.4 g/kg per day before withdrawal of CSF. The concentration of β-Amyloid was measured in the CSF before treatment and 7 - 12 days and 4 weeks after application of i.v. immunoglobulins. The results are shown in Fig. 5.

From the figure it can be seen that the amount of β-Amyloid was reduced from 1835 ng/l before treatment to 1622 ng/l (7-12 d after treatment) and 1376 ng/l (4 weeks after treatment). These results show that i.v. administration of immunoglobulins has an effect on the concentration of β-Amyloid in the CSF. Immunoglobulins also reduce β-Amyloid in the brain of patients with Alzheimer's disease.

## Claims

1. A human anti-Aβ-amyloid antibody obtained by purification from a human IgG-containing bodyfluid by Aβ-affinity chromatography.

2. Human anti-Aβ-amyloid antibody of claim 1, **characterised in that** it belongs to the class of immunoglobulines G (IgG) and does not recognise Aβ₄₀₋₁, neuropeptide F, neuropeptide Y, and Amylin, and specifically recognises one or more of Aβ₁₋₄₀,Aβ₁₋₄₂ and Aβ₂₅₋₃₅.

3. A method of purification of an anti-Aβ-amyloid antibody comprising the steps of
(i) obtaining a human IgG-containing bodyfluid,
(ii) subjecting the bodyfluid obtained to an Aβ-affinity chromatography, and
(iii) recovering the purified anti-Aβ antibody.

4. The method of claim 3 wherein the IgG-containing bodyfluid is a cerebrospinal fluid, plasma or urine obtained from one or more human beings (pooled samples).

5. The method of claim 3, wherein Aβ-affinity chromatography is carried out by an Aβ-affinity column, obtained by conjugating Aβ₁₋₄₀ onto Sepharose 4B, elution with elute buffer at pH 1.5 to 2.5 at 4°C using an FPLC system.

6. Use of an anti-Aβ-amyloid antibody according to claims 1 or 2 for treating amyloid associated diseases, especially Alzheimer's disease and primary and secondary amyloidoses.

7. Use of an anti-Aβ-amyloid antibody according to claims 1 or 2 for diagnosis of amyloid associated diseases, especially Alzheimer's disease and primary and secondary amyloidoses.

8. Use of an IgG containing, preferably IgG enriched fluid for treatment of amyloid associated diseases, especially Alzheimer's disease.

9. Pharmaceutical composition comprising an anti-Aβ-amyloid antibody according to claims 1 or 2.
